Europäisches Patentamt

(19) European Patent Office

Office européen des brevets

(11) Numéro de publication : **0 280 706 B1**

(12) **FASCICULE DE BREVET EUROPEEN**

(45) Date de publication du fascicule du brevet :
**16.10.91 Bulletin 91/42**

(51) Int. Cl.⁵ : **G06F 15/02**

(21) Numéro de dépôt : **87905623.2**

(22) Date de dépôt : **28.08.87**

(86) Numéro de dépôt international :
**PCT/FR87/00335**

(87) Numéro de publication internationale :
**WO 88/01770 10.03.88 Gazette 88/06**

(54) **DISPOSITIF POUR LA MISE EN OEUVRE D'UN PROCEDE D'ALIMENTATION DES PERSONNES.**

(30) Priorité : **01.09.86 FR 8612274**

(43) Date de publication de la demande :
**07.09.88 Bulletin 88/36**

(45) Mention de la délivrance du brevet :
**16.10.91 Bulletin 91/42**

(84) Etats contractants désignés :
**AT BE CH DE FR GB IT LI LU NL SE**

(56) Documents cités :
**WO-A-86/02194**
**US-A- 4 575 804**
**Computer Design, volume 16, No. 8, August 1977, (Concord, US), K.M. Moran: "Electricf diet controller", pages 116-118**

(73) Titulaire : **GERBAULET, Claude**
**15, rue de la Touvière**
**F-74500 Evian-les-Bains (FR)**
Titulaire : **FERRY, Sonia**
**204, rue de la Croix-Nivert**
**F-75015 Paris (FR)**

(72) Inventeur : **GERBAULET, Claude**
**15, rue de la Touvière**
**F-74500 Evian-les-Bains (FR)**
Inventeur : **FERRY, Sonia**
**204, rue de la Croix-Nivert**
**F-75015 Paris (FR)**

(74) Mandataire : **Orès, Bernard et al**
**Cabinet ORES 6, Avenue de Messine**
**F-75008 Paris (FR)**

EP 0 280 706 B1

## Description

La présente invention a pour objet un dispositif pour la mise en oeuvre d'un procédé d'alimentation des personnes.

Le dispositif de l'invention permet de conserver sa santé grâce à une alimentation saine, équilibrée et adaptée à sa morphologie, et, éventuellement, de suivre un régime amincissant ou grossissant, et cela en recommandant à une personne déterminée et pour un jour déterminé une ration énergétique qui dépend des rations énergétiques consommées par ladite personne au cours des jours précédents.

Pour mettre en oeuvre les procédés d'alimentation du type défini ci-dessus, procédés en général mis au point par des diététiciens, il existe sur le marché des balances pour aliments permettant, d'une part et de façon classique, de connaître le poids de la quantité d'aliments déposée sur le plateau, et d'autre part, de connaître l'apport énergétique de cette quantité d'aliments.

Une telle balance est divulguée, par exemple, dans US-A-4 575 804 qui décrit un dispositif comprenant un module mécano-électrique délivrant un signal électrique représentatif du poids de la quantité d'aliments déposée sur le plateau, et un module électronique susceptible de convertir ce signal afin qu'il devienne représentatif de la valeur énergétique de cette quantité d'aliments. Ce même document décrit également un calculateur de régime alimentaire permettant à un utilisateur de rapidement calculer la valeur énergétique, la teneur en hydrates de carbone et/ou la teneur en protéines des produits consommés. Cependant, si les dispositifs selon le document cité permettent d'obtenir la valeur énergétique des aliments que l'on envisage de consommer et qui sont généralement répartis en un certain nombre de catégories, les aliments d'une même catégorie ayant sensiblement la même valeur énergétique par unité de masse, une telle évaluation globale s'avère insuffisante pour une bonne hygiène d'alimentation, car elle ne tient compte ni de l'équilibre de la composition de l'alimentation, ni du fait que les effets, sur l'organisme humain, des excès alimentaires ne sont pas les mêmes selon les aliments. Ce dernier point est évidemment particulièrement important lorsqu'il s'agit de recommander une ration alimentaire pour un jour déterminé en fonction des rations alimentaires consommées au cours des jours précédents.

La présente invention vise à pallier ces inconvénients.

Elle propose, à cet effet, un dispositif pour la mise en oeuvre d'un procédé d'alimentation des personnes comprenant, en combinaison :

— des premiers moyens de mémoire pour mémoriser chaque quantité, prévue par une personne déterminée, d'aliments répartis en un nombre déterminé de catégories selon leurs valeurs énergétiques pour son alimentation au cours dudit jour,

— des deuxièmes moyens de mémoire reliés à la sortie desdits premiers moyens de mémoire pour mémoriser chaque quantité consommée par ladite personne, d'aliments de chacune desdites catégories au cours d'au moins un desdits jours précédents,

— des premiers moyens de calcul reliés à la sortie desdits deuxièmes moyens de mémoire pour calculer chaque quantité recommandée à ladite personne, d'aliments de chacune desdites catégories, ainsi que ladite ration énergétique recommandée à ladite personne en fonction des rations énergétiques consommées par ladite personne au cours des jours précédents, pour son alimentation au cours dudit jour,

— des troisièmes moyens de mémoire reliés à la sortie desdits premiers moyens de calcul pour mémoriser lesdites quantités recommandées et ladite ration énergétique recommandée,

— des deuxièmes moyens de calcul reliés à la sortie desdits premiers moyens de mémoire pour calculer la ration énergétique prévue par ladite personne pour son alimentation au cours dudit jour,

— des premiers moyens de comparaison reliés à la sortie desdits premiers moyens de mémoire à la sortie desdits deuxièmes moyens de calcul et à la sortie desdits troisièmes moyens de mémoire pour comparer chaque dite quantité prévue à chaque dite quantité recommandée de la même catégorie et pour comparer ladite ration énergétique prévue à ladite ration énergétique recommandée,

— des premiers moyens indicateurs commandés par la sortie desdits premiers moyens de comparaison, et

— des moyens de commande pour l'écriture, dans lesdits deuxièmes moyens de mémoire, desdites quantités prévues et consommées puis l'écriture, dans lesdits troisièmes moyens de mémoire des nouvelles quantités recommandées d'aliments de chacune desdites catégories, et de la nouvelle ration énergétique recommandée, pour le lendemain.

Grâce au dispositif de l'invention, il est possible de comparer, de façon simple et instantanée, les quantités d'aliments que l'on prévoit de consommer pendant ledit jour, dans chacune desdites catégories, aux quantités d'aliments recommandées qui dépendent des quantités consommées pendant les jours précédents.

Dans le cas où les quantités prévues sont en désaccord avec les quantités recommandées, on peut soit modifier les quantités prévues pour les amener en accord avec les quantités recommandées, soit passer outre, auquel cas il en est tenu compte dans le calcul des quantités recommandées pour le lendemain, ce qui permet ainsi de respecter l'équilibre de chaque catégorie sur une durée de plusieurs jours.

Les aliments étant généralement consommés au cours d'un jour en plusieurs repas comprenant chacun plusieurs plats, le dispositif de l'invention comprend, de plus, dans une réalisation avantageuse,

– des quatrièmes moyens de mémoire pour mémoriser chaque quantité prévue, par plat du prochain repas, d'aliments de chacune desdites catégories,

– des moyens de sommation, reliés à la sortie desdits quatrièmes moyens de mémoire pour sommer lesdites quantités prévues par plat d'aliments d'une même catégorie, et donc délivrer les quantités prévues, pour ledit prochain repas, d'aliments de chacune desdites catégories, et

– des cinquièmes moyens de mémoire reliés à la sortie desdits moyens de sommation pour mémoriser chaque quantité consommée au cours de l'ensemble des repas passés dudit jour, d'aliments de chacune desdites catégories, et reliés à l'entrée desdits premiers moyens de mémoire.

L'utilisation du dispositif de l'invention se trouve facilitée du fait que les quantités d'aliments sont alors mémorisées plat par plat, au moment de la préparation des repas, ce qui permet d'en corriger éventuellement la composition en connaissance de cause.

Selon une autre caractéristique avantageuse, le dispositif de l'invention comprend de plus,

– des moyens de contrôle reliés à la sortie desdits moyens de sommation et à la sortie desdits troisièmes moyens de mémoire, pour contrôler la composition dudit prochain repas, et

– des deuxièmes moyens indicateurs, commandés par lesdits moyens de contrôle.

Ces derniers comprennent, dans une réalisation avantageuse,

– des troisièmes moyens de calcul, reliés à la sortie desdits moyens de sommation, pour calculer la valeur énergétique dudit prochain repas,

– des quatrièmes moyens de calcul reliés à la sortie desdits troisièmes moyens de mémoire pour calculer une fraction déterminée de ladite ration énergétique recommandée, et

– des deuxièmes moyens de comparaison, reliés à la sortie desdits troisièmes moyens de calcul et à la sortie desdits quatrièmes moyens de calcul pour comparer ladite valeur énergétique à ladite fraction et commander lesdits deuxièmes moyens indicateurs lorsque ladite valeur énergétique est supérieure à ladite fraction.

Selon une autre caractéristique avantageuse du dispositif de l'invention, lesdits premiers moyens de calcul comprennent :

– des sixièmes moyens de mémoire des données morphologiques de ladite personne,

– des cinquièmes moyens de calcul, reliés à la sortie desdits sixièmes moyens de mémoire, pour calculer la ration énergétique journalière adaptée auxdites données morphologiques,

– des sixièmes moyens de calcul, reliés à la sortie desdits cinquièmes moyens de calcul, pour calculer chaque quantité journalière, adaptée auxdites données morphologiques, d'aliments à consommer dans chacune des catégories,

– des septièmes moyens de calcul, reliés à la sortie desdits sixièmes moyens de calcul, et à la sortie desdits deuxièmes moyens de mémorisation, pour calculer chaque dite quantité recommandée et ladite ration énergétique recommandée, et lesdits septièmes moyens de calcul sont agencés pour calculer chaque dite quantité recommandée des aliments de chacune desdites catégories en fonction, d'une part, de ladite quantité journalière adaptée de ces aliments et d'autre part, de la quantité consommée de ces aliments au cours desdits jours précédents, pris en nombre d'autant plus grand que ces aliments présentent un caractère quotidiennement indispensable.

D'autre caractéristiques et avantages de l'invention apparaîtront dans la description suivante de formes de réalisation du dispositif de l'invention, faite en se référant aux dessins annexés, sur lesquels :

– la figure 1 représente un schéma par blocs du dispositif de l'invention.

– la figure 2 représente un schéma par blocs du circuit de composition et de contrôle des repas, circuit faisant partie du dispositif de la figure 1,

– la figure 3 représente un schéma d'organisation du circuit de mémoire des quantités consommées au cours des jours précédents, circuit faisant partie du dispositif de la figure 1,

– la figure 4 représente un schéma par blocs du circuit de calculs des quantités recommandées pour le jour, circuit faisant partie du dispositif de la figure 1, et

– la figure 5 est un schéma de clavier de dispositif selon l'invention.

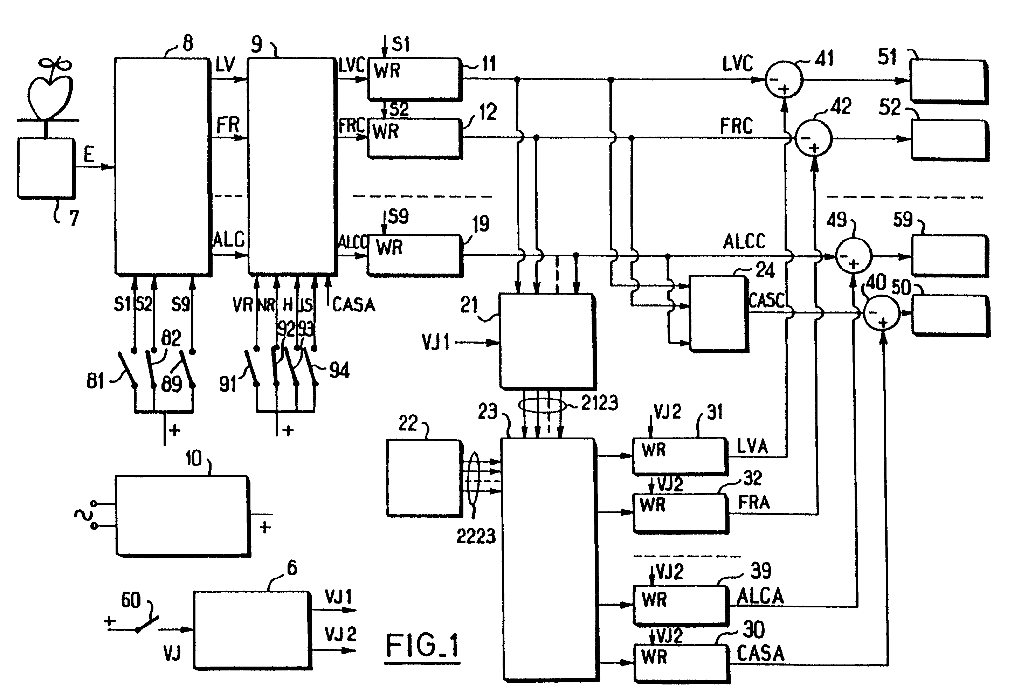

Le dispositif qui va maintenant être décrit, à titre d'exemple, en référence aux figures 1 à 4, comprend ici une balance, à l'aide de laquelle l'utilisateur du dispositif pèse les quantités d'aliments qu'il prévoit de consommer. La balance délivre, ici sous forme d'un signal électrique, des données représentatives du poids des aliments pesés. Le dispositif comprend de plus, comme cela sera vu dans la suite, des circuits électroniques de mémorisation, de calcul et d'affichage agencés pour permettre à l'utilisateur de contrôler si son alimentation

3

est saine, équilibrée et adaptée à sa morphologie, et éventuellement de suivre un régime alimentaire amincissant, grossissant ou rééquilibré.

Afin de simplifier les figures et leur compréhension, les connexions de masse et les connexions d'alimentation des différents blocs n'ont pas été représentées sur les dessins. Les autres connexions sont représentées à l'aide d'un trait unique qui symbolise alors :

– soit une connexion à un seul conducteur utilisée pour transmettre un signal analogique, ou un signal binaire, ou encore une donnée sous forme d'un signal numérique du type "série",

– soit une connexion à plusieurs conducteurs en parallèle utilisée pour transmettre une donnée sous forme d'un signal numérique du type "parallèle".

Les catégories d'aliments prises en compte ici sont au nombre de neuf. Le tableau I indique brièvement la définition de chacune de ces catégories, l'abréviation symbolique qui leur sera associée au cours de cette description, et la valeur énergétique approximative de cent grammes de ces aliments (dans la suite, l'unité de masse utilisée pour peser les aliments est le gramme).

| Catégorie | Abréviation | Valeur énergétique / 100 g |
|---|---|---|
| Légumes verts | LV | 40 cal |
| Fruits frais | FR | 50 cal |
| Céréales, féculents, pain | FC | 300 cal |
| Viande, poisson, oeufs | VP | 160 cal |
| Laitages, fromages frais | LA | 80 cal |
| Fromages secs | FM | 250 cal |
| Beurre, huile, margarine | BH | 600 cal |
| Sucre, configure, miel | SU | 400 cal |
| Vin, bière | ALC | 70 cal |

**TABLEAU I**

Naturellement les catégories ci-dessus et les valeurs énergétiques correspondantes ne sont données qu'à tire d'exemple et il est bien entendu possible de choisir un nombre plus faible, ou au contraire, un nombre plus élevé de catégories, ou de définir les catégories à partir de critères nutritionnels autres que ceux utilisés dans l'exemple ci-dessus. En outre, les catégories alimentaires de qualité nutritionnelle équivalente peuvent être regroupées sous une même catégorie (viande/poisson/oeufs de l'exemple ci-dessus) ou éclatées en catégories distinctes (légumes vers/fruits frais, ou laitages, fromages frais,/fromages secs de l'exemple ci-dessus) pour des raisons de présentation... Ces catégories distinctes peuvent éventuellement être couplées au niveau du traitement et des préconisations de l'appareil.

En se référant maintenant à la figure 1, la balance 7, ici commercialisée par la Société TERRAILLON (capteurs SCAIME) sous la référence BE 5PZ, délivre en sortie un signal numérique E représentatif du poids, donc de la quantité des aliments déposés sur le plateau dont elle est pourvue.

Un multiplexeur 8 est pourvu d'une entrée recevant le signal numérique E, d'autant de sorties que de catégories d'aliments, ici neuf, délivrant des signaux numériques LV, FR,... ALC, et de neuf entrées de commande recevant neuf signaux binaires $S_1$ à $S_9$.

Les signaux binaires $S_1$ à $S_9$ sont produits à l'aide de neuf interrupteurs 81 à 89, reliés par une extrémité à une tension correspondant au niveau logique haut, tension produite par un circuit d'alimentation de type connu 10 relié à une source d'énergie électrique, pile, batterie rechargeable, ou secteur, et servant également à alimenter tous les circuits du dispositif, de façon non représentée car classique, comme cela a déjà été signalé.

Le multiplexeur 8, de type connu, est agencé pour relier l'entrée recevant le signal E à la sortie délivrant le signal LV lorsque le signal $S_1$ est au niveau haut, à la sortie délivrant le signal FR lorsque le signal $S_2$ est au niveau haut, et ainsi de suite...

Un circuit 9 de composition et de contrôle des repas, qui sera décrit plus en détail dans la suite, est ici

4

pourvu de neuf entrées recevant les signaux LV, FR,... ALC, de neuf sorties délivrant des signaux numériques LVC, FRC... ALCC, de quatre entrées de commande recevant quatre signaux binaires VR, NR, H et JS, et d'une dixième entrée recevant un signal numérique CASA.

Les signaux binaires VR, NR, H et JS sont produits à l'aide de quatre interrupteurs 91 à 94, reliés par une extrémité à la tension correspondant au niveau logique haut.

Neuf registres-mémoires 11 à 19, de type connu, sont chacun pourvus d'une entrée recevant les signaux LVC, FRC, ... ALCC respectivement, d'une sortie délivrant ces mêmes signaux numériques LVC, FRC, .... ALCC respectivement, d'une sortie délivrant ces mêmes signaux numériques LVC, FRC, ..., ALCC respectivement, et d'une entrée de commande d'écriture recevant les signaux $S_1$, $S_2$, ... $S_9$ respectivement.

Un circuit de mémoire 21 des quantités consommées au cours des jours précédents, qui sera décrit plus en détail dans la suite est pouvu de neuf entrées recevant les signaux LVC, FRC, ..., ALCC, d'une pluralité de sorties regroupées en un faisceau 2123, et d'une entrée de commande d'écriture recevant un signal binaire VJ1.

Un circuit de calcul 24 est pourvu de neuf entrées recevant les signaux LVC, FRC, ..., ALCC, et d'une sortie délivrant un signal numérique CASC. Le circuit de calcul 24 est agencé pour multiplier le signal présent sur chacune des entrées par un coefficient déterminé et pour additionner les neufs produits ainsi obtenus. A titre d'exemple, le circuit 24 calcule ici :

CASC=0,4LVC+0,5FRC+3FCC+1,6VPC+0,8LAC+2,5FMC+6BHC+4SUC+0,7ALCC

Le circuit 24 est donc à la portée d'un homme de métier et ne sera pas décrit davantage.

Dix soustracteurs 40 à 49, de type connu, sont pourvus chacun d'une entrée moins recevant les signaux CASC, LVC, FRC, ...ALCC, respectivement, d'une entrée plus recevant des signaux numériques CASA, LVA, FRA, ... ALCA respectivement, et d'une sortie délivrant un signal numérique.

Dix indicateurs 50 à 59 sont reliés chacun à la sortie des dix soustracteurs 40 à 49, respectivement. Chaque indicateur comprend ici un dispositif d'affichage alphanumérique de type connu, pour afficher la valeur numérique du signal présent à son entrée.

Un circuit de mémoire 22 des données morphologiques de l'utilisateur, de type connu, est pourvu ici d'une pluralité de sorties, regroupées en un faisceau 2223, délivrant chacune un signal numérique représentatif de la morphologie de l'utilisateur.

A titre d'exemple, ces signaux sont ici au nombre de cinq et comprennent :
– le signal T représentant la taille
– le signal NS représentant le sexe
– le signal PR représentant le poids
– le signal VO représentant la morphologie générale de l'utilisateur longiligne, intermédiaire ou bréviligne
– le signal F représentant l'activité physique de l'utilisateur, faible, moyenne ou intense.

Toujours à titre d'exemple, les valeurs numériques mémorisées dans le circuit de mémoire 22 sont données par le tableau II.

| T | taille en centimètres |
|---|---|
| NS | 4 pour un homme <br> 2 pour une femme |
| PR | poids en kilogrammes |
| VO | 1 morphologie longiligne <br> 1,05 morphologie intermédiaire <br> 1,10 morphologie bréviligne |
| F | 1 activité physique faible (sédentaire) <br> 2 activité physique moyenne <br> 3 activité physique intense |

TABLEAU II

Un circuit de calcul 23, qui sera décrit plus en détail dans la suite, est pourvu d'une première pluralité d'entrées reliées au faisceau 2223, d'une deuxième pluralité d'entrées reliées au faisceau 2123 et de dix sorties délivrant chacune un signal numérique.

Dix registres-mémoires 30 à 39, de type connu, sont chacun pourvus d'une entrée reliée à chacune des dix sorties du circuit de calcul 23, d'une sortie délivrant les signaux numériques CASA, LVA, ..., ALCA respectivement et d'une entrée de commande d'écriture recevant un signal binaire VJ2.

Un circuit électronique 6, de commande d'écriture, est pourvu d'une entrée recevant un signal binaire VJ, produit à l'aide d'un interrupteur 60 relié par une extrémité à la tension correspondant au niveau logique haut, et de deux sorties délivrant les signaux binaires VJ1 et VJ2. Le circuit électronique 6 est agencé pour que, lorsque le signal VJ passe au niveau haut, les signaux binaires VJ1 et VJ2 commandent l'écriture dans la mémoire 21 et dans les registres 30 à 39, respectivement, l'écriture dans les registres 30 à 39 étant légèrement retardée par rapport à l'écriture dans la mémoire 21. Le circuit 6 est donc à la portée de l'homme de métier et ne sera pas davantage décrit.

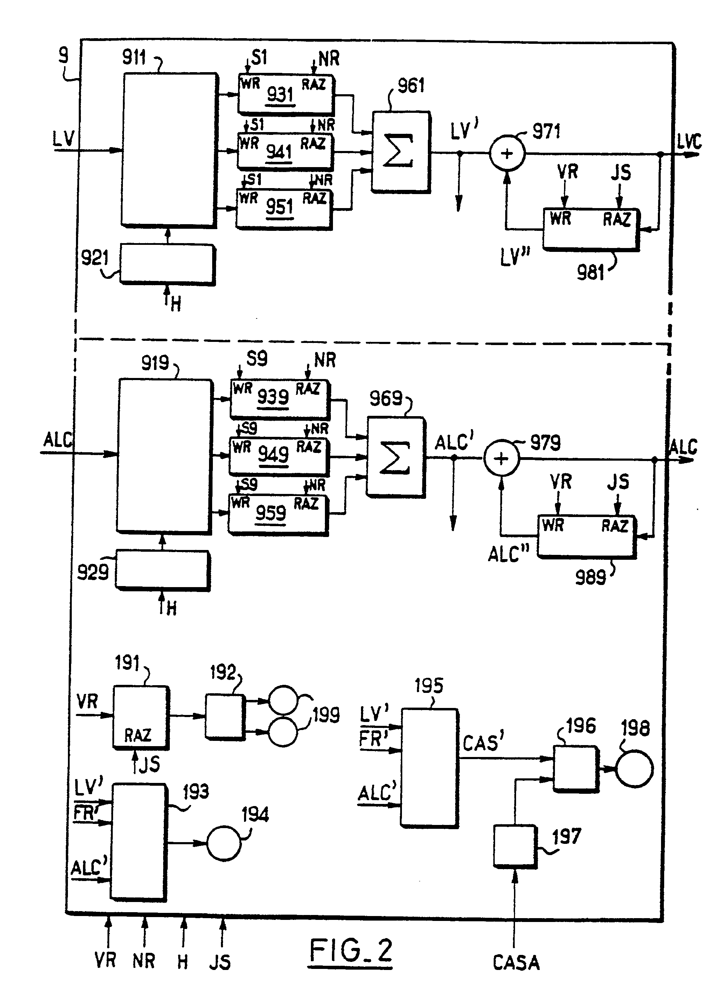

En référence à la figure 2, le circuit 9 de composition et de contrôle des repas est maintenant décrit. Ce circuit comprend d'abord neuf voies identiques, reliant les neufs entrées recevant les signaux LV, FR, ... ALC aux neuf sorties délivrant les signaux LVC, FRC, ... ALCC, respectivement. Afin de ne pas alourdir la figure, on n'a représenté que les voies de traitement des signaux LV et ALC.

La voie de traitement du signal LV comprend un multiplexeur 911 de type connu, pourvu d'une entrée recevant le signal LV, d'ici trois sorties reliées à trois registres-mémoires 931, 941 et 951, et d'une entrée de commande reliée à la sortie d'un compteur 921.

Le compteur 921, est du type connu qui compte, modulo 3, les passages au niveau haut du signal binaire H, qu'il reçoit sur son entrée de comptage. Ainsi, si le multiplexeur 911 est dans l'état où l'entrée recevant le signal LV est reliée au registre mémoire 931, un passage au niveau haut du signal H commande un changement d'état du multiplexeur 911 pour relier l'entrée recevant le signal LV au registre mémoire 941, et ainsi de suite.

Chaque registre mémoire 931, 941 et 951, de type connun, est pourvu d'une entrée de commande d'écriture recevant le signal $S_1$, d'une entrée de commande de remise à zéro recevant le signal NR, et d'une sortie. Les trois sorties des registres mémoires 931, 941 et 951 sont reliées aux trois entrées d'un sommateur 961, dont la sortie délivre un signal numérique LV'.

Un additionneur 971 est pourvu d'une première entrée recevant le signal LV', d'une deuxième entrée recevant un signal LV" de sortie d'un registre mémoire 981, et d'une sortie, reliée à l'entrée du registre mémoire 981, et délivrant le signal LVC.

Le registre mémoire 981, de type connu, est pourvu d'une entrée de commande d'écriture recevant le signal VR et d'une entrée de commande de remise à zéro recevant le signal JS.

La voie de traitement du signal FR se déduit de la précédente en remplaçant, dans chaque chiffre de référence, le nombre des unités, valant 1, par le nombre 2. Ainsi, c'est le signal $S_2$ qui est reçu par l'entrée de commande d'écriture des registres mémoires 932, 942 et 952.

Il en va de même pour les autres voies.

Le circuit 9, de composition et de contrôle des repas comprend également un circuit de calcul 195, pourvu de neuf entrées recevant les signaux LV', FR', ... ALC' et d'une sortie délivrant un signal numérique CAS'. Le circuit de calcul 195 est identique au circuit de calcul 24 déjà décrit.

Un circuit de calcul 197, pourvu d'une entrée recevant le signal CASA, et d'une sortie, est agencé pour multiplier ledit signal par une fraction inférieure à 1, ici égale à 7/10.

Un comparateur numérique 196, de type connu, pourvu d'une première entrée recevant le signal CAS', d'une deuxième entrée recevant le signal de sortie du circuit 197, et d'une sortie binaire reliée à un voyant indicateur 198, commande ici l'éclairage du voyant si le signal appliqué sur sa première entrée est supérieur au signal appliqué sur sa deuxième entrée.

Un circuit électronique 193 est pourvu de neuf entrées recevant les signaux LV', FR', ... ALC' et d'une sortie binaire reliée à un voyant indicateur 194. Le circuit électronique 193 est ici agencé pour détecter la combinaison suivante des signaux :
LV'=FR'=VP'=LA'=FM'=O FC',BH',SU',ALC' quelconques et commander l'éclairage du voyant 194. Le circuit de détection 193 est à la portée de l'homme de métier et ne sera pas davantage décrit.

Un compteur 191, de type connu, est pourvu d'une entrée de comptage recevant le signal VR, d'une entrée de commande de remise à zéro recevant le signal JS et d'une sortie délivrant un signal numérique représentant le nombre de passages au niveau haut du signal VR.

Un circuit de comparaison 192 est pourvu d'une entrée reliée à la sortie du compteur 191 et de deux sorties binaires reliées chacune à un voyant indicateur 199. Le circuit de comparaison 192 est agencé pour comparer la valeur numérique à son entrée aux bornes d'une plage de nombres, ici la plage 3, 4, 5 et 6 et pour commander un des voyants indicateurs 199 si ce nombre est inférieur à 3 et l'autre voyant indicateur si ce nombre est supérieur à 6. Le circuit 192 est à la portée de l'homme de métier et il ne sera pas décrit davantage.

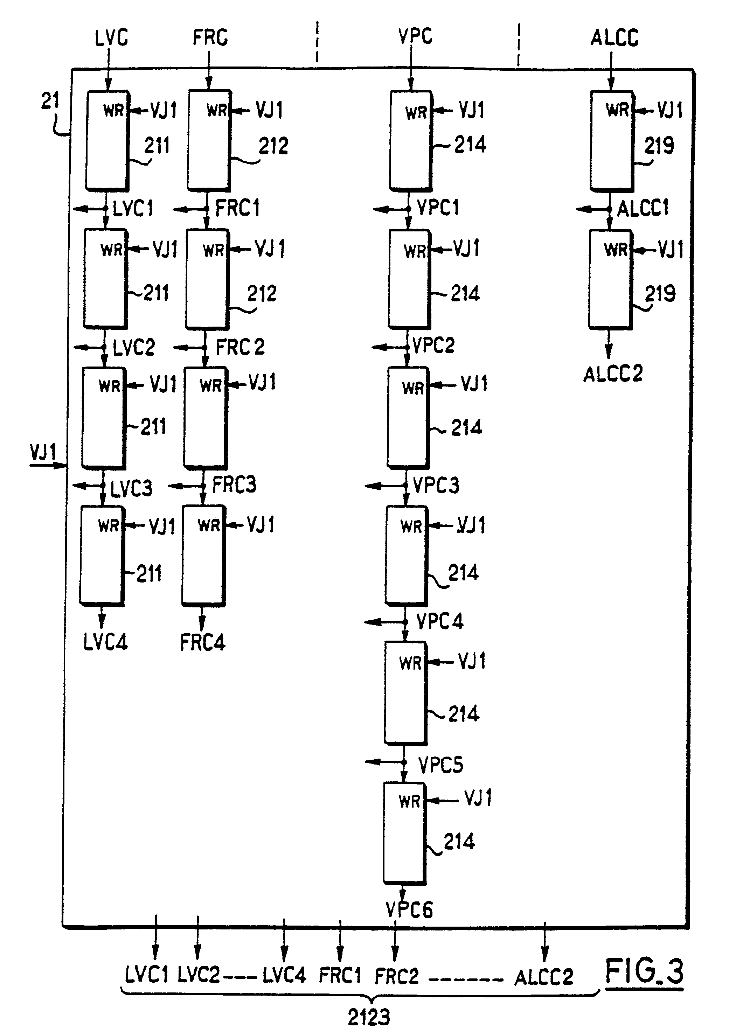

L'organisation du circuit 21 de mémoire des quantités consommées au cours des jours précédents est maintenant décrite, en référence à la figure 3.

Le circuit de mémoire 21 est ici organisé en neuf chaînes de registres mémoires 211 à 219 de type connu, montés en cascades à l'intérieur d'une chaîne.

Les registres mémoires sont tous pourvus d'une entrée de commande d'écriture recevant le signal binaire VJ1.

Chaque chaîne de registres 211 à 219 fait suite à une des neuf entrées LVC, FRC, ... ALCC. Le nombre de registres d'une chaîne dépend de l'entrée à laquelle elle fait suite. A titre d'exemple, le tableau III indique le nombre de registres dans chaques chaînes.

| Chaîne | Référence des registres | Nombre de registres |
|---|---|---|
| LVC | 211 | 4 |
| FRC | 212 | 4 |
| FCC | 213 | 4 |
| VPC | 214 | 6 |
| LAC | 215 | 6 |
| FMC | 216 | 6 |
| BHC | 217 | 2 |
| SUC | 218 | 2 |
| ALCC | 219 | 2 |

TABLEAU III

La sortie de chacun des registres 211 à 219 est reliée au faisceau 2123. La sortie d'un registre d'une chaîne délivre un signal numérique désigné par le nom du signal de la chaîne suivi du rang du registre dans la chaîne. Ainsi le troisième registre 214 délivre le signal VPC3. Sur la figure 3, seules les chaînes LVC, FRC, VPC et ALCC ont été représentées, afin de ne pas alourdir la figure.

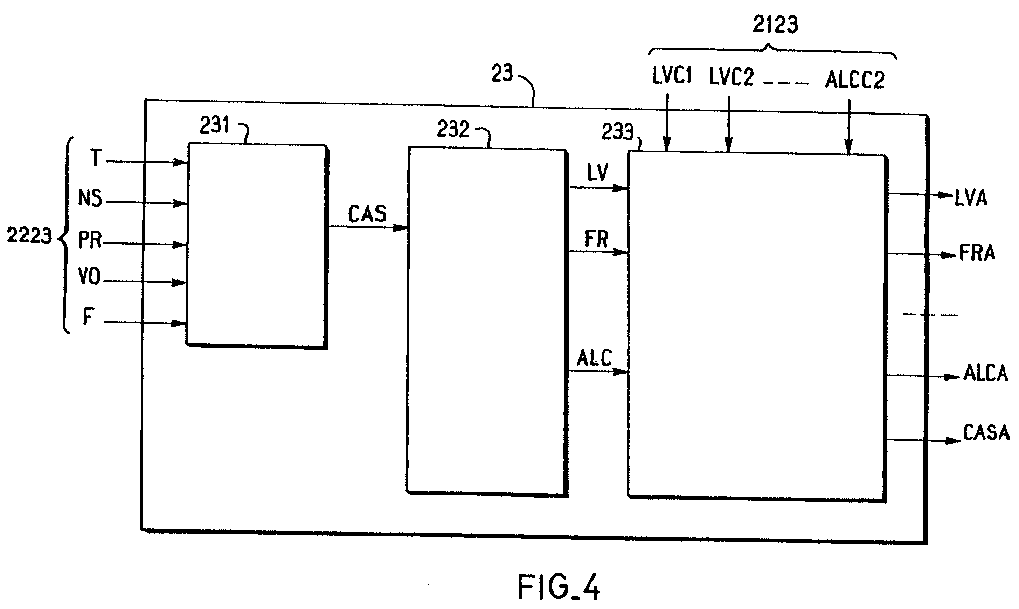

Le circuit de calcul 23 est maintenant décrit en référence à la figure 4.

Un circuit 231 de calcul de la ration énergétique journalière adaptée est pourvu d'une pluralité d'entrées recevant le faisceau 2223, et d'une sortie délivrant le signal CAS. Le circuit 231 effectue un certain nombre d'opérations simples sur les signaux numériques qu'il reçoit. Ainsi, et à titre d'exemple, dans le cas où ces signaux sont les signaux T, NS, PR, UO et F, le circuit 231 effectue les calculs qui suivent.

Le poids idéal PID est d'abord calculé :

$$PID = VO\ [T-100-(T-150)\ /\ NS]$$

puis la ration énergétique journalière idéale CAI :

$$CAI = 2000 + 25\ (PID-50) + 500\ (P-1)$$

puis la différence pondérale DP :

$$DP = 100\ (PR-PID)\ /\ PID$$

puis le coefficient QN entre la ration énergétique journalière adaptée et la ration énergétique journalière idéale :

$$QN = 1 - 2\ DP\ /\ 100$$

(limité vers le bas à 0,6 et vers le haut à 1)

et enfin la ration énergétique CAS journalière adaptée :

$$CAS = CAI \times QN$$

(limitée vers le bas à 1200 cal.)

Le circuit de calcul 231 est donc à la portée d'un homme de métier et il ne sera pas décrit davantage.

Un circuit 232 de calcul des quantités journalières adaptées d'aliments de chaque catégorie est pourvu d'une entrée recevant le signal CAS et de neuf sorties délivrant des signaux numériques LV, FR, ... ALC. Le circuit 232 calcule des fonctions du signal numérique CAS qu'il reçoit.

A titre de premier exemple, le circuit 232 effectue ici les calculs suivants (Système d'équations du premier degré) :

$$LV = 0,1\ .\ CAS + 400$$
$$FR = 0,15.\ CAS + 80$$
$$FC = 0,13.\ CAS - 150$$
$$VP = 0,06.\ CAS + 220$$
$$LA = 0,024.CAS + 250$$
$$FM = 0,02.\ CAS + 20$$
$$BH = 0,03.\ CAS - 30$$
$$SU = 0,02.\ CAS - 25$$
$$ALC = 0,1\ .\ CAS - 100$$

Le circuit de calcul 232 est donc à la portée d'un homme de métier et il ne sera pas décrit davantage.

A titre de deuxième exemple, le circuit 232 peut effectuer les calculs suivants (Système d'équations du second degré et du premier degré) :

$$LV = -0,0000090\ .\ CAS\ .\ CAS + 0,135\ .\ CAS + 250$$
$$FR = -0,0000060\ .\ CAS\ .\ CAS + 0,090\ .\ CAS + 165$$
$$FC = -0,0000091\ .\ CAS\ .\ CAS + 0,200\ .\ CAS - 175$$
$$VP = \qquad\qquad\qquad\qquad\qquad + 0,060\ .\ CAS + 220$$

$$LA = +0,0000075\ .\ CAS\ .\ CAS - 0,015\ .\ CAS + 140$$
$$FM = +0,0000025\ .\ CAS\ .\ CAS - 0,005\ .\ CAS + 50$$
$$BH = -0,0000020\ .\ CAS\ .\ CAS + 0,033\ .\ CAS - 40$$
$$SU = +0,0000080\ .\ CAS\ .\ CAS - 0,012\ .\ CAS + 15$$
$$ALC = \qquad\qquad\qquad\qquad\qquad + 0,100\ .\ CAS - 100$$

Un circuit 233 de calcul des quantités recommandées et de la ration énergétique recommandée est pourvu de neuf entrées recevant les signaux LV, FR, ... ALC, d'une pluralité d'entrées reliées au faisceau 2123, et de dix sorties délivrant les signaux LVA, FRA, ... ALCA et CASA. Le circuit 233 effectue des opérations simples sur les signaux numériques qu'il reçoit. A titre d'exemple, le circuit 233 effectue ici les calculs suivants :

$$LVA = 2\,LV - (LVC1 + LVC2 + LVC3 + LVC4) / 4$$
$$FRA = 2\,FR - (FRC1 + FRC2 + FRC3 + FRC4) / 4$$
$$FCA = 2\,FC - (FCC1 + FCC2 + FCC3 + FCC4) / 4$$
$$VPA = 2\,VP - (VPC1 + VPC2 + VPC3 + VPC4 + VPC5 + VPC6) / 6$$
$$LAA = 2\,LA - (LAC1 + LAC2 + LAC3 + LAC4 + LAC5 + LAC6) / 6$$
$$FMA = 2\,FM - (FMC1 + FMC2 + FMC3 + FMC4 + FMC5 + FMC6) / 6$$
$$BHA = 2\,BH - (BHC1 + BHC2) / 2$$
$$SUA = 2\,SU - (SUC1 + SUC2) / 2$$
$$ALCA = 2\,ALC - (ALCC1 + ALCC2) / 2$$
$$CASA = 0{,}4\,LVA + 0{,}5\,FRA + 3\,FCA + 1{,}6\,VPA + 0{,}8\,LAA + 2{,}5\,FMA + 6\,BHA + 4\,SUA + 0{,}7\,ALCA$$

Le circuit de calcul 233 est donc à la portée d'un homme de métier et il ne sera pas décrit davantage.

Le dispositif qui vient d'être décrit fonctionne comme suit.

L'utilisateur commence tout d'abord par rentrer ses données morphologiques dans la mémoire 22, d'une façon non décrite car classique, par exemple à l'aide des touches d'un clavier.

Le circuit 231 calcule alors la ration énergétique journalière CAS adaptée à ces données morphologiques.

Le circuit 232 calcule ensuite, à partir de la ration énergétique journalière CAS, les quantités journalières d'aliments LV, FR, ... ALC, adaptées à ces données morphologiques, dans chacune des catégories d'aliments.

Ensuite, lors de la première mise en service du dispositif, il est prévu une procédure d'initialisation de la mémoire 21, effectuée à l'aide de moyens connus, et non représentés sur les figures dans un souci de simplicité. Au cours de cette procédure d'initialisation, la quantité LV est inscrite dans tous les registres 211, la quantité FR dans tous les registres 212, et ainsi de suite. Comme cela apparaîtra plus clairement dans la suite, ceci revient à supposer que l'utilisateur s'est nourri de façon idéale au cours des jours précédant la première mise en service du dispositif.

Le dispositif est alors prêt pour une utilisation quotidienne, qui ne deviendra totalement efficace qu'au bout d'une semaine environ, lorsque seront prises en compte les quantités d'aliments réellement absorbées par l'utilisateur au cours de cette période.

Avant de composer le premier plat du premier repas de la première journée, l'utilisateur manoeuvre l'interrupteur 92 et l'interrupteur 93, les signaux NR et H passent au niveau haut, les registres 931 à 939, 941 à 949 et 951 à 959 sont mis à zéro et les multiplexeurs 911 à 919 sont dans l'état où leur entrée est reliée aux registres 931 à 939 respectivement.

L'utilisateur dépose ensuite sur le plateau de la balance 7, catégorie d'aliments par catégorie d'aliments, les différentes quantités qui composent le premier plat du premier repas.

Lorsqu'il pèse des légumes verts, l'utilisateur manoeuvre l'interrupteur S1, ce qui a pour effet d'inscrire dans le registre 931 le poids, représentatif de la quantité de légumes verts prévus pour le premier plat. Lorsqu'il pèse des fruits, il manoeuvre S2, ce qui inscrit dans le registre 932 (non représenté sur les figures) la quantité de fruits prévus et ainsi de suite jusqu'à ce que la quantité d'alcool se trouve inscrite dans le registre 939.

Pour passer à la composition du deuxième plat, l'utilisateur manoeuvre l'interrupteur 93, ce qui fait passer le signal H au niveau haut et commande aux multiplexeurs 911 à 919 de relier leur entrée aux registres 941 à 949 respectivement. Les aliments sont pesés comme pour le premier plat, et les quantités s'inscrivent alors dans les registres 941 à 949.

La composition du troisième plat s'effectue de même, étant entendu qu'on ne s'est limité à trois plats dans la présente description que dans un souci de ne pas la compliquer inutilement.

Ainsi, on peut dire que les interrupteurs 92, 93, 81 à 89, les compteurs 921 à 929, les multiplexeurs 911 à 919 et les registres 931 à 939, 941 à 949 et 951 à 959 permettent de mémoriser chaque quantité prévue, par plat du repas, des aliments de chaque catégorie.

Les sommateurs 961 à 969 délivrent donc des signaux LV', FR', ... ALC' représentatifs des quantités prévues pour le repas, des aliments de chaque catégorie.

Le circuit de calcul 195 calcule alors le signal CAS' qui est représentatif de la valeur énergétique du repas. Si cette valeur est supérieure aux sept dixièmes de CASA, CASA représentant, comme cela sera vu plus clairement dans la suite, la ration énergétique journalière recommandée, le voyant 198 s'allume, indiquant que le repas est trop copieux.

Si le circuit de détection 193 détecte l'absence simultanée d'aliments des catégories LV, FR, VP, LA et FM, le voyant 194 s'allume, indiquant que le repas est déséquilibré.

On peut donc dire que le circuit de détection 193, les circuits de calcul 195 et 197, et le comparateur 196 contrôlent la composition du repas.

9

Si aucun des voyants 194 et 198 ne s'allume, l'utilisateur va alors, en principe, valider son repas en manoeuvrant l'interrupteur 91. Si un des voyants 194 et 198 s'allume, l'utilisateur peut revoir la composition de son repas s'il le désire, de façon à le rendre moins copieux, ou plus équilibré, ou les deux. Mais il peut également passer outre et valider ce repas mal composé, s'il est décidé à le consommer, en manoeuvrant l'interrupteur 91.

La manoeuvre de l'interrupteur 91 entraîne l'activation du signal VR et donc l'inscription, dans les registres 981 à 989, des valeurs LV"=LV', FR"=FR', ... ALC"=ALC' car la sortie des registres 981 à 989 était initialement à zéro.

Lorsque l'utilisateur veut préparer son deuxième repas de la journée, il manoeuvre l'interrupteur 92, ce qui a pour effet la remise à zéro des registres 931 à 939, 941 à 949 et 951 à 959.

Les étapes de la composition du deuxième repas sont évidemment identiques à celles de la composition du premier repas.

Lorsque l'utilisateur décide de valider son deuxième repas, il manoeuvre l'interrupteur 91, ce qui entraîne l'inscription, dans les registres 981 à 989, des valeurs des signaux LV", FR", ... ALC" qui sont maintenant représentatives des quantités d'aliments consommées au cours des deux premiers repas, alors que les valeurs des signaux LV', FR', ... ALC' représentent les quantités consommées au cours du deuxième repas.

L'utilisateur procède de même pour les repas suivants. Les signaux LV", FR", ... ALC" sont donc représentatifs des quantités consommées au cours de l'ensemble des repas passés dudit jour.

L'interrupteur 91, les additionneurs 971 à 979 et les registres 981 à 989 permettent donc la mémorisation des signaux LV",FR",... ALC" représentatifs de ces quantités.

Après avoir prévu la composition de son dernier repas de la journée, et avant la validation de ce repas, les indicateurs 50 à 59 indiquent à l'utilisateur les différences entre les quantités prévues pour la journée LVC, FRC, ... ALCC, respectivement et les quantités recommandées LVA, FRA, ... ALCA, respectivement, calculées comme cela sera vu dans la suite.

L'utilisateur peut soit modifier la composition de son dernier repas, pour arriver à des quantités prévues en accord avec les quantités recommandées, soit passer outre et valider le dernier repos, en manoeuvrant l'interrupteur 91, puis valider la journée, en manoeuvrant l'interrupteur 60.

De même, en fin de journée, les voyants 199 indiquent à l'utilisateur si le nombre de ses repas a été trop faible c'est-à-dire ici inférieur à 3, ou au contraire trop grand, c'est-à-dire ici supérieur à 6.

Au moment où le signal VJ1 est activé par le circuit 6, les quantités LVC, FRC, ... ALCC, qui représentent alors les quantités consommées au cours de la journée passée sont écrites dans les premiers registres 211 à 219, le contenu des premiers registres passant alors dans les seconds registres, et ainsi de suite, le contenu des registres situés au bout de chaîne se trouvant perdu.

Ainsi les quantités LVC1, FRC1, ... ALC1 représentent les quantités consommées au cours de la journée passée, les quantités LVC2, FRC2, ... ALC2 celles consommées au cours de la journée précédente et ainsi de suite...

Le circuit de calcul 233 calcule alors les quantités recommandées pour la journée à venir, LVA, FRA, ... ALCA, et la ration calorique recommandée CASA à l'aide des formules déjà indiquées à titre d'exemple. Dans ces formules, on notera qu'on ne tient pas compte de façon identique des consommations des journées passées pour tous les aliments.

Ainsi, les neuf catégories d'aliments sont ici réparties en trois groupes, selon leur caractère quotidiennement indispensable. Le premier groupe comprend les catégories VP, LA et FM regroupant des aliments que l'on peut considérer comme quasiment indispensables quotidiennement, le deuxième groupe comprend les catégories LV, FR et FC, dont la quantité indispensable tolère de larges fluctuations et le troisième groupe les catégories BH, SU et ALC, dont on peut rester privé longtemps, voire en permanence, sans véritable préjudice pour la santé.

Le circuit de calcul 233 calcule les quantitées recommandées pour la journée à venir en fonction des quantités consommées les jours précédents, ces jours étant pris en nombre d'autant plus grand que ces aliments présentent un caractère quotidiennement indispensable.

Ainsi, la consommation des aliments du premier groupe est ici moyennée sur sept jours, celle des aliments du deuxième groupe sur cinq jours, et celle des aliments du troisième groupe sur trois jours.

Chaque quantité recommandée est celle qui rendrait la consommation, sur la période de moyennage, égale à la consommation adaptée aux données morphologiques.

Les quantités recommandées LVA, FRA, ... ALCA, et la ration énergétique recommandée CASA, calculées par les circuits 22 et 23, sont écrites dans les registres 31 à 39, et 30 respectivement, lors de l'activation du signal VJ2 par le circuit 6.

Le dispositif est alors prêt pour une nouvelle journée, que l'utilisateur commence en manoeuvrant l'interrupteur 94, de façon à activer le signal JS pour remettre à zéro le compteur 191 et les registres 981 à 989.

Le dispositif de l'invention peut évidemment être réalisé à l'aide de composants électroniques discrets, mais également à l'aide d'un microprocesseur et de ses circuits associés.

Naturellement, la présente invention n'est pas limitée à la description qui vient d'être faite.

En particulier, les calculs effectués par les circuits 231, 232 et 233 mettent en oeuvre des formules utilisées en diététique. Ces formules sont susceptibles d'être changées sans que cela remette en cause le dispositif de l'invention, en fonction par exemple des principes diététiques utilisés, ou du régime particulier auquel peut être soumis l'utilisateur.

De même, les circuits de soustraction 40 à 49, qui servent en fait à comparer les quantités prévues LVC, FRC, .... ALCC, aux quantités recommandées LVA, FRA, ... ALCA et la ration énergétique prévue CASC à la ration énergétique recommandée CASA pourraient être remplacés par de simples comparateurs, et les dispositifs d'affichage 50 à 51 pourraient être remplacés par de simples voyants indicateurs.

Il est également à la portée d'un homme de métier de prévoir la possibilité de tenir compte d'un plat donné, un plat tout préparé par exemple, non pas à partir du poids de ses constituants, mais à partir de la proportion en ses différents constituants, en référence aux catégories alimentaires disponibles sur le dispositif.

Naturellement, le dispositif de l'invention étant un dispositif électronique, il est possible de le doter d'un certain nombre de perfectionnements à la portée de l'homme de métier, destinés à en rendre l'utilisation plus agréable, comme, par exemple :

– horloge indiquant le jour et l'heure,

– possibilité de comparer, à tout moment, les quantités d'aliments prévues aux quantités d'aliments adaptées,

– remise à zéro totale ou partielle du circuit de mémoire des quantités consommées les jours précédents.

– Possibilité de programmer à volonté le dispositif suivant de théories nutritionnelles autres que celle exprimée par l'algorithme contenu dans l'appareil, au moyen de touches de programmation ou d'éléments externes tels que cartes à mémoire, PROMS ou autres moyens de mémorisation.

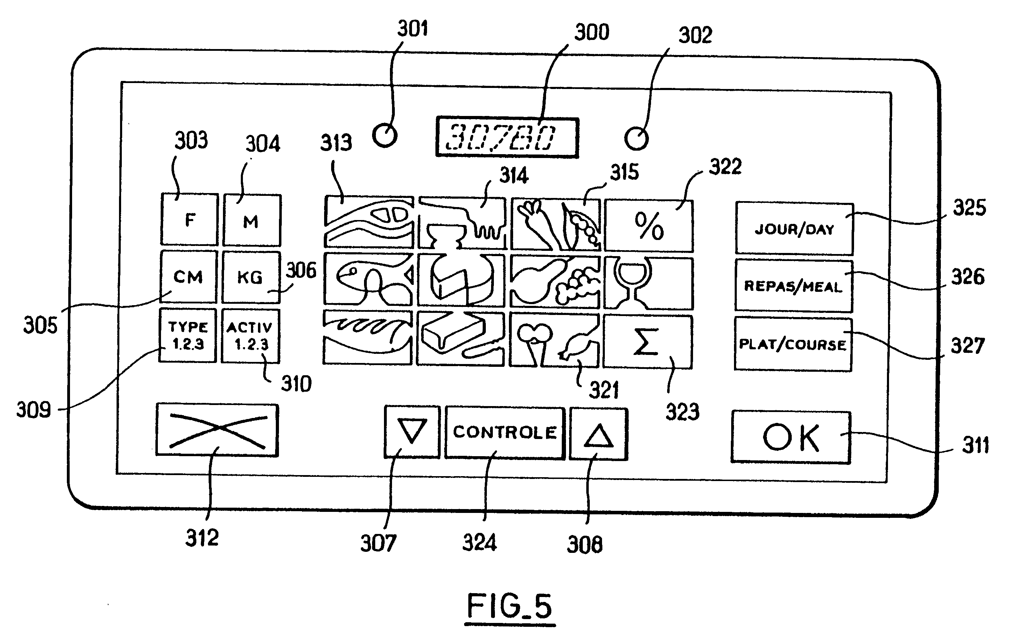

L'invention prévoit également une réalisation sans balance associée, sous forme d'un dispositif de faible encombrement dont le clavier et la façade sont montrés sur la figure 5. Dans cette réalisation, la face frontale du boîtier enfermant les éléments électroniques décrits ci-dessus comprend un moyen d'affichage 300 par exemple du type LCD flanqué par deux voyants 301 et 302, rouge et vert respectivement. Des touches 303 et 304 permettent de sélectionner le sexe de l'utilisateur, des touches 305 et 306 d'afficher sa taille et son poids en actionnant les touches de commande 307 et 308 lesquelles servent également à la saisie des caractéristiques morphologiques et d'activité de l'utilisateur repérées par des touches 309 et 310.

Une touche 311, marquée OK, valide les saisies effectuées tandis qu'une touche 312 est prévue pour l'annulation.

Les catégories d'aliments sont représentées schématiquement sur les neuf touches 313 - 321 ; une touche 322 est marquée "%", une touche 323 est marquèe "Σ", une touche 324 est marquée "contrôle" et trois touches 325-327 sont marquées, respectivement, JOUR, REPAS, PLAT.

Après manoeuvre d'un interrupteur marche-arrêt les caractéristiques de l'utilisateur saisies à l'aide des touches 303-310 sont validées par appui sur la touche 311.

De même, les catégories d'aliments sont saisies à l'aide des touches 313 - 321, la touche 322 étant utilisée pour la saisie d'un aliment dons la valeur énergétique s'écarte de la valeur moyenne de la catégorie, en se référant à une table d'équivalence et en manoeuvrant les touches 307 et 308.

En fin de saisie, un appui sur la touche 311 valide l'opération. Si l'appui sur cette touche est concomitant à l'appui sur la touche 327, la validation a lieu pour le plat, ou pour le repas, si la touche utilisée est la touche 326, ou pour le jour si la touche enfoncée est celle montrée en 325.

Pour la saisie d'un plat complexe, on actionne d'abord la touche 323 "Σ" puis l'appui sur les touches 307 et 308 permet d'afficher le poids total du plat, on actionne alors la touche 322 puis une touche 313-321 et à nouveau les touches 307 et 308 pour ajuster le pourcentage du poids total représenté par un premier ingrédient, puis la touche de validation 311, puis une nouvelle touche 313-321, etc... jusqu'à validation finale du plat par appui sur les touches 311 et 323 ou 311 et 327.

Pour le contrôle de l'état de la ration autorisée, un appui sur la touche 324 et sur une des touches 313-321 provoque l'affichage de la quantité en grammes encore autorisée pour le jour en cours de la catégorie d'aliment correspondant à la touche enfoncée. Simultanément, l'allumage du voyant 301, indique que toute la ration autorisée a été consommée si la lueur est fixe, tandis qu'un allumage clignotant indique qu'il reste moins de 25 % de la ration autorisée. Inversement, un allumage fixe du voyant 302 traduit qu'il reste plus de 25 % de la ration autorisée et un allumage clignotant est signe d'une carence.

Pour un contrôle de la situation globale, on provoque un appui sur les touches 324 et 323 : celui-ci déclenche l'allumage du voyant 301 si une des rations a été totalement consommée ou l'allumage du voyant 302 en cas de carence d'un aliment.

## Revendications

1. Dispositif pour la mise en oeuvre d'un procédé d'alimentation des personnes comprenant, en combinaison :

– des premiers moyens de mémoire (11-19, 81-89) pour mémoriser chaque quantité (LVC, FRC, ... ALCC), prévue par une personne déterminée, d'aliments répartis en un nombre déterminé de catégories selon leurs valeurs énergétiques pour son alimentation au cours dudit jour,

– des deuxièmes moyens de mémoire (21) reliés à la sortie desdits premiers moyens de mémoire (11-19), pour mémoriser chaque quantité (LVC1, LVC2, ..., LVC4, FRC1, FRC2, ..., ALCC2), consommée par ladite personne, d'aliments de chacune desdites catégories au cours d'au moins un desdits jours précédents,

– des premiers moyens de calcul (22, 23), reliés à la sortie desdits deuxièmes moyens de mémoire (21), pour calculer chaque quantité (LVA, FRA, ..., ALCA), recommandée à ladite personne, d'aliments de chacune desdites catégories, ainsi que ladite ration énergétique (CASA) recommandée à ladite personne en fonction des rations énergétiques consommées par ladite personne au cours des jours précédents, pour son alimentation au cours dudit jour,

– des troisièmes moyens de mémoire (30-39), reliés à la sortie desdits premiers moyens de calcul (22, 23), pour mémoriser lesdites quantités recommandées (LVA, FRA, ..., ALCA) et ladite ration énergétique recommandée (CASA),

– des deuxièmes moyens de calcul (24), reliés à la sortie desdits premiers moyens de mémoire (11-19) pour calculer la ration énergétique (CASC), prévue pour ladite personne, pour son alimentation au cours dudit jour,

– des premiers moyens de comparaison (40-49) reliés à la sortie desdits premiers moyens de mémoire (11-19), à la sortie desdits deuxièmes moyens de calcul (24) et à la sortie desdits troisièmes moyens de mémoire (30-39), pour comparer chaque dite quantité prévue (LVC, FRC, ..., ALCC) à chaque dite quantité recommandée (LVA, FRA, ..., ALCA) de la même catégorie, et pour comparer ladite ration énergétique prévue (CASC) à ladite ration énergétique recommandée (CAS),

– des premiers moyens indicateurs (50-59), commandés par la sortie desdits premiers moyens de comparaison (40-49), et

– des moyens de commande (6, 60) pour l'écriture, dans lesdits deuxièmes moyens de mémoire (21), desdites quantités prévues et consommées (LVC, FRC, ..., ALCC) puis l'écriture, dans lesdits troisièmes moyens de mémoire (30-39) des nouvelles quantités recommandées d'aliments de chacune desdites catégories, et de la nouvelle ration énergétique recommandée, pour le lendemain.

2. Dispositif selon la revendication 1, comprenant de plus,

– des quatrièmes moyens de mémoire (911-919, 921-929, 931-939, 941-949, 951-959, 93, 81-89, 92) pour mémoriser chaque quantité prévue, par plat d'un repas, d'aliments de chacune desdites catégories,

– des moyens de sommations (961-969), reliés à la sortie desdits quatrièmes moyens de mémoire (931-939, 941-949, 951-959) pour sommer lesdites quantités prévues par plat d'aliments d'une même catégorie, et donc délivrer les quantités prévues (LV', FR', ... ALC'), pour ledit repas, d'aliments de chacune desdites catégories, et

– des cinquièmes moyens de mémoire (971-979, 981-989, 91) reliés à la sortie desdits moyens de sommation (961-969) pour mémoriser chaque quantité consommée (LV", FR",... ALC") au cours de l'ensemble des repas passés d'un jour, d'aliments de chacune desdites catégories, et reliés à l'entrée desdits premiers moyens (11-19) de mémoire.

3. Dispositif selon la revendication 2, comprenant de plus;

– des moyens de contrôle (193, 195-197) reliés à la sortie desdits moyens de sommation (961-969) et à la sortie desdits troisièmes moyens de mémoire (30), pour contrôler la composition d'un repas, et

– des deuxièmes moyens indicateurs (194, 198), commandés par lesdits moyens de contrôle (193, 195-197).

4. Dispositif selon la revendication 3, dans lequel lesdits moyens de contrôle comprennent :

– des troisièmes moyens de calcul (195), reliés à la sortie desdits moyens de sommation (961-969), pour calculer la valeur calorique (CAS') d'un repas,

– des quatrièmes moyens de calcul (197), reliés à la sortie des dits troisièmes moyens de mémoire (30) pour calculer une fraction déterminée de ladite ration énergétique recommandée (CASA),

– des deuxièmes moyens de comparaison (196) reliés à la sortie desdits troisièmes moyens de calcul (195) et à la sortie desdits quatrièmes moyens de calcul (197) pour comparer ladite valeur énergétique (CAS') à ladite fraction et commander lesdits deuxièmes moyens indicateurs (198) lorsque ladite valeur énergétique (CAS') est supérieure à ladite fraction.

5. Dispositif selon l'une des revendication 3 et 4, dans lequel lesdits moyens de contrôle comprennent des moyens (193), reliés à la sortie desdits moyens de sommation (961-969), pour détecter l'absence d'aliments de catégories déterminées et pour commander lesdits deuxièmes moyens indicateurs (194) en cas de détection.

6. Dispositif selon l'une des revendications 1 à 5, comprenant de plus :

– des moyens de comptage (91, 191) pour compter le nombre de repas au cours d'un jour,

– des troisièmes moyens de comparaison (192), reliés à la sortie desdits moyens de comptage (91, 191) pour comparer ledit nombre de repas aux bornes d'une plage de nombres déterminée, et

– des troisièmes moyens (199) indicateurs, reliés à la sortie desdits deuxièmes moyens de comparaison (192), commandés lorsque ledit nombre de repas est extérieur à ladite plage de nombres.

7. Dispositif selon l'une des revendications 1 à 6, dans lequel lesdits premiers moyens de calcul comprennent :

– des sixièmes moyens de mémoire (22) des données morphologiques (T, NS, PR, UO, F) de ladite personne,

– des cinquièmes moyens de calcul (231), reliés à la sortie desdits sixièmes moyens de mémoire (22), pour calculer la ration énergétique (CAS) journalière adaptée auxdites données morphologiques (T, NS, PR, UO, F),

– des sixièmes moyens de calcul (232), reliés à la sortie desdits cinquièmes moyens de calcul (231), pour calculer chaque quantité (LV, FR,..., ALC) journalière, adaptée auxdites données morphologiques (T, NS, PR, UO, F) d'aliments à consommer dans chacune des catégories,

– des septièmes moyens de calcul (233), reliés à la sortie desdits sixièmes moyens de calcul (232), et à la sortie desdits deuxièmes moyens de mémoire (21) pour calculer chaque dite quantité recommandée (LVA, FRA, ... ALCA) et ladite ration énergétique recommandée (CASA).

8. Dispositif selon la revendication 7, dans lequel, lesdits septièmes moyens de calcul (233) sont agencés pour calculer chaque dite quantité recommandée (LVA, FRA, ... ALCA) des aliments de chacune desdites catégories en fonction, d'une part, de ladite quantité journalière adaptée (LV, FR,... ALC) de ces aliments et d'autre part, de la quantité consommée (LVC1+LVC2+LVC3+LVC4, FRC1+FRC2, ..., ALC1+ALC2) de ces aliments au cours desdits jours précédents, pris en nombre d'autant plus grand que ces aliments présentent un caractère quotidiennement indispensable.

9. Dispositif selon la revendication 7, dans lequel lesdits sixièmes moyens de calcul (232) sont agencés pour calculer chaque dite quantité journalière adaptée (LV, FR, ... ALC), à partir de ladite ration énergétique journalière adaptée (CAS) et à l'aide, pour chacune desdites catégories, d'une fonction de ladite ration énergétique journalière adaptée (CAS).

10. Dispositif selon l'une des revendications 1 à 9, comprenant en outre :

– une balance (7) pour aliments, délivrant un signal (E) représentatif du poids de la quantité d'aliments pesée, et

– des moyens de multiplexage (8, 81-89), reliés à la sortie de ladite balance (7) et à l'entrée desdits moyens de mémoire (11,19).

11. dispositif selon l'une des revendication 1 à 9, caractérisé en ce qu'il enferme dans un boîtier les éléments constitutifs des moyens de mémoire, de calcul, de comparaison et de commande, et en ce que ledit boîtier présente sur une de ses faces un clavier à touches d'actionnement desdits moyens ainsi que des organes de visualisation des données saisies et des informations fournies.


## Patentansprüche

1. Einrichtung zum Durchführen eines Verfahrens zum Ernähren von Personen, die in Kombination folgendes aufweist:

– erste Speichermittel (11-19, 8-89) zum Speichern jeder für eine bestimmte Person vorgesehenen Menge (LVC, FRC, ... ALCC) von Nahrungsmitteln, die in einer bestimmten Anzahl von Kategorien nach ihren Nährwerten zum Zuführen derselben im Laufe des Tages aufgeteilt sind,

– zweite Speichermittel (21), die mit dem Ausgang der ersten Speichermittel (11-19) verbunden sind, um jede von der Person im Laufe zumindest eines der vorhergehenden Tage konsumierte Menge (LVC1, LVC2, ..., LVC4, FRC1, FRC2, ..., ALCC2) von Nahrungsmitteln jeder der Kategorien zu speichern,

– erste Rechnermittel (22, 23), die mit dem Ausgang der zweiten Speichermittel (21) verbunden sind, um jede der Person empfohlene Menge (LVA, FRA, ..., ALCA) von Nahrungsmitteln jeder der Kategorien sowie die Energieration (CASA), welche der Person in Abhängigkeit von von der Person im Laufe der vorhergehenden Tage konsumierten Energierationen empfohlen ist, zum Zuführen derselben im Laufe des Tages zu berechnen,

– dritte Speichermittel (30-39), die mit dem Ausgang der ersten Rechnermittel (22, 23) verbunden sind, um die empfohlenen Mengen (LVA, FRA, ..., ALCA) und die empfohlene Energieration (CASA) zu speichern,

– zweite Rechnermittel (24), die mit dem Ausgang der ersten Speichermittel (11-19) verbunden sind, um die für die Person vorgesehene Energieration (CASC) zum Zuführen derselben im Laufe des Tages zu berechnen,

– erste Vergleichsmittel (40-49), die mit dem Ausgang der ersten Speichermittel (11-19), mit dem Ausgang der zweiten Rechnermittel (24) und mit dem Ausgang der dritten Speichermittel (30-39) verbunden sind, um jede vorgesehene Menge (LVC, FRC, ..., ALCC) mit jeder empfohlenen Menge (LVA, FRA, ..., ALCA) derselben Kategorie zu vergleichen und um die vorgesehene Energieration (CASC) mit der empfohlenen Energieration (CAS) zu vergleichen,

– erste Anzeigemittel (50-59), die von dem Ausgang der ersten Vergleichsmittel (40-49) angesteuert werden und

– Bedienungsmittel (6, 60) zum Schreiben der vorgesehenen und konsumierten Mengen (LVC, FRC, ..., ALCC) in die zweiten Speichermittel (21), sodann zum Schreiben der neuen empfohlenen Menge von Nahrungsmitteln jeder der Kategorien und der neuen empfohlenen Energieration in die dritten Speichermittel (30-39) für den folgenden Tag.

2. Einrichtung nach Anspruch 1, die außerdem folgendes aufweist:

– vierte Speichermittel (911-919, 921-929, 931-939, 941-949, 951-959, 93, 81-89, 92), um jede durch ein Gericht für ein Mahl vorgesehene Menge von Nahrungsmitteln jeder der Kategorien zu speichern,

– Summierungsmittel (961-969), die mit dem Ausgang der vierten Speichermittel (931-939, 941-949, 951-959) verbunden sind, um die durch ein Gericht von Nahrungsmitteln einer gleichen Kategorie vorgesehenen Mengen zu summieren und folglich die vorgesehenen Mengen (LV', FR', ..., ALC') von Nahrungsmitteln jeder der Kategorien für das Mahl zu liefern und

– fünfte Speichermittel (971-979, 981-989, 91), die mit dem Ausgang der Summierungsmittel (961-969) verbunden sind, um jede, im Laufe der Folge der eingenommenen Mahle eines Tages konsumierte Menge (LV", FR", ... ALC") von Nahrungsmitteln jeder der Kategorien zu speichern und die mit dem Eingang der ersten Speichermittel (11-19) verbunden sind.

3. Einrichtung nach Anspruch 2, die außerdem folgendes aufweist:

– Prüfungsmittel (193, 195-197), die mit dem Ausgang der Summierungsmittel (961-969) und mit dem Ausgang der dritten Speichermittel (30) verbunden sind, um die Zusammenstellung eines Mahles zu prüfen und

– zweite Anzeigemittel (194, 198), die von den Prüfungsmitteln (193, 195-197) angetrieben werden.

4. Einrichtung nach Anspruch 3, bei welcher die Prüfungsmittel folgendes aufweisen:

– dritte Rechnermittel (195), die mit dem Ausgang der Summierungsmittel (961-969) verbunden sind, um den Kalorienwert (CAS') eines Mahles zu berechnen,

– vierte Rechnermittel (197), die mit dem Ausgang der dritten Speichermittel (30) verbunden sind, um einen bestimmten Bruchteil der empfohlenen Energieration (CASA) zu berechnen,

– zweite Vergleichsmittel (196), die mit dem Ausgang der dritten Rechnermittel (195) und mit dem Ausgang der vierten Rechnermittel (197) verbunden sind, um den Näherwert (CAS') mit dem Bruchteil zu vergleichen und die zweiten Anzeigemittel (198) anzutreiben, wenn der Nährwert (CAS') größer ist, als der Bruchteil.

5. Einrichtung nach einem der Ansprüche 3 und 4, bei welcher die Prüfungsmittel Mittel (193) aufweisen, die mit dem Ausgang der Summierungsmittel (961-969) verbunden sind, um das Fehlen von Nahrungsmitteln bestimmter Kategorien zu erfassen und um die zweiten Anzeigemittel (194) im Falle einer Erfassung anzusteuern.

6. Einrichtung nach einem der Ansprüche 1 bis 5, die außerdem folgendes aufweist:

– Zählungsmittel (91, 191) zum Zählen der Anzahl von Mahlen im Laufe eines Tages,

– dritte Vergleichsmittel (192), die mit dem Ausgang der Zählungsmittel (91, 191) verbunden sind, um die Anzahl von Mahlen mit den Grenzen eines bestimmten Bereichs für die Anzahl zu vergleichen und

– dritte Anzeigemittel (199), die mit dem Ausgang der zweiten Vergleichsmittel (192) verbunden sind, die angesteuert werden, wenn die Anzahl von Mahlen größer ist als der Bereich für die Anzahl.

7. Einrichtung nach einem der Ansprüche 1 bis 6, bei welcher die ersten Rechnermittel folgendes aufweisen:

– sechste Speichermittel (22) für die morphologischen Daten (T, NS, PR, UO, F) der Person,

– fünfte Rechnermittel (231), die mit dem Ausgang der sechsten Speichermittel (22) verbunden sind, um die tägliche Enerieration (CAS) zu berechnen, welche den morphologischen Daten (T, NS, PR, UO, F) angepaßt ist,

– sechste Rechnermittel (232), die mit dem Ausgang der fünften Rechnermittel (231) verbunden sind, um

14

jede tägliche den morphologischen Daten (T, NS, PR, UO, F) angepaßte Menge (LV, FR, ..., ALC) von Nahrungsmitteln zu berechnen, die in jeder der Kategorien konsumiert werden sollen,

– siebente Rechnermittel (233), die mit dem Ausgang der sechsten Rechnermittel (232) und mit dem Ausgang der zweiten Speichermittel (21) verbunden sind, um jede empfohlene Menge (LVA, FRA, ..., ALCA) und die empfohlene Energieration (CASA) zu berechnen.

8. Einrichtung nach Anspruch 7, bei welcher die siebenten Rechnermittel (233) eingerichtet sind, um jede empfohlene Menge (LVA, FRA, ..., ALCA) von Nahrungsmitteln jeder der Kategorien in Abhängigkeit einerseits von der angepaßten täglichen Menge (LV, FR, ... ALC) dieser Nahrungsmittel und andererseits von der konsumierten Menge (LVC1+LVC2+LVC3+LVC4, FRC1+FRC2, ..., ALC1+ALC2) dieser Nahrungsmittel im Laufe der vorhergehenden Tage zu berechnen, die in der Anzahl umso größer genommen werden, als diese Nahrungsmittel eine täglich unentbehrliche Eigenart darstellen.

9. Einrichtung nach Anspruch 7, bei welcher die sechsten Rechnermittel (232) eingerichtet sind, um jede angepaßte, tägliche Menge (LV, FR, ... ALC) von der angepaßten täglichen Energieration (CAS) an und mit HJilfe einer Funktion der angepaßten täglichen Energieration (CAS) für jede der Kategorien zu berechnen.

10. Einrichtung nach einem der Ansprüche 1 bis 9, die außerdem folgendes aufweist:

– eine Waage (7) für Nahrungsmittel, die ein Signal (E) liefert, welches das Gewicht der gewogenen Menge von Nahrungsmitteln repräsentiert und

– Multiplexmittel (8, 81-89), die mit dem Ausgang der Waage (7) und mit dem Eingang der Speichermittel (11, 19) verbunden sind.

11. Einrichtung nach einem der Ansprüche 1 bis 9, die dadurch gekennzeichnet ist, daß sie in einem Gehäuse die Bauelemente der Speicher-, der Rechner-, der Vergleichs- und der Antriebsmittel einschließt und daß das Gehäuse auf einer seiner Seiten ein Tastenfeld zum Betätigen der Mittel sowie Anzeigeteile für die Erfassungsdaten und die gelieferten Informationen aufweist.

## Claims

1. Device for implementing a method for feeding persons comprising, in combination :

– first memory means (11-19, 81-89) to memorize each quantity (LVC, FRC, ..., ALCC), anticipated by a given person, of foods subdivided in a given number of categories according to their energy values, for his/her diet during that day ;

– second memory means (21) connected to the outlet of said first memory means (11-19) to memorize each quantity (LVC1, LVC2, ..., LVC4, FRC1, FRC2, ... ALCC2), as consumed by said person, of food belonging to each of said categories during at least one of said preceding days ;

– first computer means (22, 23), connected to the outlet of said second memory means (21), to determine each quantity (LVA, FRA, ..., ALCA) of food in each of said categories which is recommended for said person, as well as said energy ration (CASA), recommended to said person, for his/her diet during said day, as a function of the energy rations consumed by said person during the preceding days ;

– third memory means (30-39), connected to the outlet of said first computer means (22, 23), to memorize said recommended quantities (LVA, FRA, ..., ALCA) and said recommended energy ration (CASA) ;

– second computer means (24), connected to the outlet of said first memory means (11-19) to determine the energy ration (CASC), as anticipated for said person, for his/her diet during said day ;

– first comparison means (40-49) connected to the outlet of said first memory means (11-19), to the outlet of said second computer means (24) and to the outlet of said third memory means (30-39), to compare each of said anticipated quantities (LVC, FRC, ..., ALCC) to each said recommended quantity (LVA, FRA,..., ALCA) in the same category, and to compare said anticipated energy ration (CASC) to said recommended energy ration (CAS) ;

– first indicator means (50-59), controlled by the outlet of said first comparison means (40-49), and

– controlling means (6, 60) for writing, in said second memory means (21), said antiticpated and consumed quantities (LVC, FRC, ..., ALCC) and then writing, in said third memory means (30-39), new recommended quantities of food in each of said categories, and the new recommended energy ration for the next day.

2. A device according to claim 1, further comprising :

– fourth memory means (911-919, 921-929, 931-939, 941-949, 951-959, 93, 81-89, 92) to memorize each anticipated quantity, per course in a meal, of food from each of said categories ;

– summing up means (961-969), connected to the outlet of said fourth memory means (931-939, 941-949, 951-959) to sum up said anticipated quantities, per course, of food from the same category and thus deliver anticipated quantities (LV', FR', ..., LC'), for said meal, of food from each of said categories, and

– fifth memory means (971-979, 981-989, 91) connected to the outlet of said summing up means (961-969) to memorize each consumed quantity (LV″, FR″,...,ALC″) during all past meals of said day, of food from each of said categories, and connected to the inlet of said first memory means (11-19).

3. A device according to claim 2, further comprising :
– monitoring means (193, 195-197) connected to the outlet of said summing up means (961-969) and to the outlet of said third memory means (30), to monitor the composition of a meal, and
– second indicator means (194, 198) controlled by said monitoring means (193, 195-197).

4. A device according to claim 3, in which said monitoring means include :
– third computing means (195), connected to the outlet of said summing up means (961-969), to determine the energy value (CAS′) of a meal ;
– fourth computing means (197), connected to the outlet of said third memory means (30) to determine a given fraction of said recommended energy ration (CASA) ;
– second comparison means (196) connected to the outlet of said third computing means (195) and to the outlet of said fourth computing means (197) for comparing said energy value (CAS′) with said fraction and controlling said second indicator means (198) when said energy value (CAS′) is superior to said fraction.

5. A device according to one of claims 3 and 4, in which said monitoring means include means (193) connected to the outlet of said summing up means (961-969), for detecting the absence of food from given categories and for controlling said second indicator means (194) in case of detection.

6. A device according to one of claims 1 to 5, further comprising :
– computing means (91, 191) to determine the number of meals during a day ;
– third comparison means (192) connected to the outlet of said computing means (91, 191) to compare said number of meals to the limits of a given number interval, and
– third indicator means (199) connected to the outlet of said second comparison means (192), controlled when said number of meals is outside said number interval.

7. A device according to one of claims 1 to 6, in which said first computing means include :
– sixth memory means (22) of said person's morphological data (T, NS, PR, UO, F),
– fifth computing means (231), connected to the outlet of said sixth memory means (22), to determine the daily energy ration (CAS) adapted to said morphological data (T, NS, PR, UO, F),
– sixth computing means (232), connected to the outlet of said fifth computing means (231), to determine each daily quantity (LV, FR,...,ALC), adapted to said morphological data (T, NS, PR, UO, F) of food to be consumed in each category ;
– seventh computing means (233) connected to the outlet of said sixth computer means (232) and to the outlet of said second memory means (21) to determine each said recommended quantity (LVA, FRA,...,ALCA) and said recommended energy ration (CASA).

8. A device according to claim 7, in which said seventh computing means (233) are arranged so as to determine each said recommended quantity (LVA, FRA,...,ALCA) of food from each of said categories as a function, on the one hand, of said adapted daily quantity (LV, FR,...,ALC) of these foods and on the other hand, of the quantity (LVC1+LVC2+LVC3+LVC4, FRC1+FRC2,..., ALC1+ALC2) of these foods consumed during said preceding days, taken in all the greater number as these foods must absolutely be taken every day.

9. A device according to claim 7, in which said sixth computing means (232) are arranged so as to determine each said adapted daily quantity ( LV, FR,...,ALC) from said adapted daily energy ration (CAS) and with the help, for each of said categories, of a function of said adapted daily energy ration (CAS).

10. A device according to one of claims 1 to 9, further comprising :
– a food scale (7), delivering a signal (E) representative of the weight of the quantity of food weighted, and
– multiplexing means (8, 81-89), connected to the outlet of said scale (7) and to the inlet of said memory means (11, 19).

11. A device according to one of claims 1 to 9, characterized in that it includes in a housing the constituent elements of the memory means, the computing means, the comparison means, and the controlling means, and in that said housing has on one of its sides a keyboard with keys for activating said means, as well as display elements for entered data and given information.

FIG.1

FIG_2

FIG.3

EP 0 280 706 B1

FIG.4

**FIG_5**